# EUROPÄISCHE PATENTSCHRIFT

(11) **EP 3 873 422 B1**
(45) Veröffentlichungstag und Bekanntmachung des Hinweises auf die Patenterteilung: **19.02.2025**
(21) Anmeldenummer: 19797677.2
(22) Anmeldetag: 31.10.2019
(51) Int. Cl.: A61K 8/58, A61K 8/31, A61K 8/34, A61Q 5/00

(54) **ORGANISCHE SILIZIUMVERBINDUNGEN IN EINER WASSERFREIEN PHASE ZUR ERHÖHUNG VON DEREN LAGERSTABILITÄT**
ORGANIC SILICON COMPOUNDS IN AN ANHYDROUS PHASE TO INCREASE THEIR STORAGE STABILITY
COMPOSÉS ORGANIQUES DU SILICIUM EN PHASE ANHYDRE POUR AUGMENTER LEUR STABILITÉ AU STOCKAGE

(30) Priorität: 31.10.2018 DE 102018127290
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE) (DE); SCHULZE ZUR WIESCHE, Erik, 33619 Bielefeld (DE)
(86) Internationale Anmeldenummer: PCT/EP2019/079776
(87) Internationale Veröffentlichungsnummer: WO 2020/089362

(56) Entgegenhaltungen:
- WO-A1-2013/117449
- WO-A1-2017/102857
- US-A1- 2010 303 748
- MOJUN ZHU ET AL: "How To Prepare Reproducible, Homogeneous, and Hydrolytically Stable Aminosilane-Derived Layers on Silica", LANGMUIR, vol. 28, no. 1, 30 November 2011 (2011-11-30), US, pages 416 - 423, XP055650371, ISSN: 0743-7463, DOI: 10.1021/la203638g

## Beschreibung

Die vorliegende Erfindung betrifft ein wasserfreies Trägermedium zur Erhöhung der Lagerstabilität von organischen Siliciumverbindungen, wie in den Ansprüchen beschrieben.

Die äußere Beanspruchung der Haare durch chemische Stoffe aus einer Vielzahl unterschiedlicher Quellen stellt die Entwicklung kosmetischer Pflegeprodukte vor Herausforderungen. Luft- und Wasserverunreinigungen wirken sich nachteilig auf Haut und Haare aus. Die Wirkung verschiedener Luftschadstoffe kann in Gegenwart anderer Luftschadstoffe und unter Einwirkung von UV-Strahlung verstärkt werden.

Es ist bekannt, dass die Toxizität von gasförmigen Schadstoffen der Luft, wie Schwefeldioxid, Ozon und Stickoxiden, insbesondere mit ihrer Initiatoraktivität für freie Radikale zusammenhängt, die bei Lebewesen Schäden verursachen. Freie Radikale sind Stoffwechselprodukte, die auch natürlicherweise im Körper vorkommen. In dem Fall spricht man von "oxidativem Schaden". Freie Radikale können auch eine Haarschädigung bewirken, die beispielsweise als Verringerung des Glanzes sowie des Griffs und/oder des Verblassens der Haarfarbe sichtbar wird.

Ferner sind oft wechselnde Konsumentenwünsche hinsichtlich einer bestimmten Beschaffenheit der Haare mit einer wiederkehrenden chemischen Beanspruchung der Haare verbunden. Beispielsweise beanspruchen Haarfärbungen die Haare, aufgrund dessen eine besondere, intensive Pflege nötig sein kann.

Zum Schutz und zur Pflege der Haare werden im Stand der Technik siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann.

Das Problem bei der Verwendung von siliciumorganischen Verbindungen ist deren Instabilität gegenüber Wasser. Wässrige Systeme zur Haarbehandlung sind somit nachteilbehaftet, wenn sie organische Silicumverbindungen als Wirksubstanzen enthalten.

Die der vorliegenden Erfindung zugrunde liegende Aufgabe besteht in der Bereitstellung eines Mediums, das die Lagerstabilität von organischen Siliciumverbindungen erhöht. Insbesondere lag der vorliegenden Erfindung die Aufgabe zugrunde, ein wasserfreies Trägermedium bereitzustellen, das organische Siliciumverbindungen stabilisiert, um für die Herstellung eines anwendungsbereiten kosmetischen Mittels bereit zu stehen.

Diese Aufgabe wird gelöst durch die Merkmalskombination in Anspruch 1.

Das Trägermedium dient zur Bereitstellung einer siliciumorganischen Verbindung. In dem Trägermedium soll die organische Siliciumverbindung laberstabil bleiben. Das Trägermedium dient dann zur Kombination mit einer oder mehreren weiteren Zusammensetzungen, die für die Haarpflege dienende Wirkstoffe enthält und auf Wasser basieren kann. Dadurch dass das Trägermedium dann mit der einen oder den weiteren Zusammensetzungen erst kurz vor der Anwendung zusammengegeben werden kann, bleibt die Wirksubstanz siliciumorganische Verbindung bis zur Anwendung stabil.

Unter wasserfrei soll im Rahmen der vorliegenden Erfindung bevorzugt verstanden werden, dass dem Trägermedium nicht Wasser zugegeben wird oder das Trägermedium nicht wasserbasiert ist. Bevorzugter liegt der Wassergehalt des wasserfreien Trägermediums bei weniger als 5 Gew.-%, noch bevorzugter bei weniger als 2 Gew.-%, am meisten bevorzugt bei weniger als 1 Gew.-%, bezogen auf das Gesamtgewicht des wasserfreien Trägermediums. Beim Vorliegen von geringen Feuchtigkeitsmengen kann ein geringer Teil der organischen Siliciumverbindung hydrolysieren und das Hydrolysat liegt im Gleichgewicht mit freiem Wasser vor. Diese Menge an Wasser liegt bevorzugt in den oben genannten Mengen vor. Derartige Zusammensetzungen finden sich z.B. in US 2010/303748 A1, (HERCOUET LEILA [FR], 2. Dezember 2010 (2010-12-02).

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden. Als ersten erfindungswesentlichen Bestandteil enthält das wasserfreie Trägermedium zur Erhöhung der Lagerstabilität von organischen Siliciumverbindungen mindestens eine organische Siliciumverbindung, nämlich die eine oder die mehreren Verbindungen, die stabilisiert werden sollen. Bevorzugte organische Siliciumverbindungen werden ausgewählt aus Silanen mit einem, zwei oder drei Siliciumatomen, wobei die organische Siliciumverbindung eine oder mehrere Hydroxylgruppen und/oder hydrolysierbare Gruppen pro Molekül umfasst.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die organische Siliciumverbindungen sind Verbindungen, die ein bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das wasserfreie Trägermedium zur Erhöhung der Lagerstabilität enthält mindestens eine organischen Siliciumverbindung, die bevorzugt aus Silanen mit einem, zwei oder drei Siliciumatomen ausgewählt ist, wobei die organische Siliciumverbindung eine oder mehrere Hydroxylgruppen oder hydrolysierbare Gruppen pro Molekül umfasst.

Im Rahmen einer ganz besonders bevorzugten Ausführungsform weist das wasserfreie Trägermedium zur Erhöhung der Lagerstabilität mindestens eine organische Siliciumverbindung auf, die aus Silanen mit einem, zwei oder drei Siliciumatomen ausgewählt ist, wobei die organische Siliciumverbindung außerdem eine oder mehrere basische Gruppe und eine oder mehrere Hydroxylgruppen oder hydrolysierbare Gruppen pro Molekül umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Bei der oder den hydrolysierbaren Gruppen handelt es sich bevorzugt um eine C₁-C₆-Alkoxygruppe, insbesondere um eine Ethoxygruppe oder um eine Methoxygruppe. Es ist bevorzugt, wenn die hydrolysierbare Gruppe direkt an das Siliciumatom gebunden vorliegt. Handelt es sich beispielsweise bei der hydrolysierbaren Gruppe um eine Ethoxygruppe, so enthält die organische Siliciumverbindung bevorzugt eine Struktureinheit R'R"R‴Si-O-CH₂-CH₃. Die Reste R', R" und R‴ stellen hierbei die drei übrigen freien Valenzen des Siliciumatoms dar.

Ganz besonders gute Ergebnisse konnten erhalten werden, wenn das wasserfreie Trägermedium zur Behandlung eines keratinischen Materials mindestens eine organische Siliciumverbindung der Formel (I) und/oder (II) enthält.

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

In einer weiteren ganz besonders bevorzugten Ausführungsform enthält das wasserfreie Trägermedium mindestens eine organische Siliciumverbindung der Formel (I) und/oder (II),

R₁R₂N-L-Si(OR₃)ₐ(R₄)_{b} (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht,

   (R₅O)_{c}(Rₑ)_{d}Si-(A)ₑ-[NR₇-(A')]_{f}-[O-(A")]_{g}-[NR₈-(A‴)]ₕ-Si(R₆')_{d'}(OR₅')_{c'} (II),

   wobei
   - R5, R5', R5" unabhängig voneinander für ein Wasserstoffatom oder für eine C₁-C₆-Alkylgruppe stehen,
   - R6, R6' und R6" unabhängig voneinander für eine C₁-C₆-Alkylgruppe stehen,
   - A, A', A", A‴ und Aʺʺ unabhängig voneinander für eine lineare oder verzweigte, zweibindige C₁-C₂₀-Alkylengruppe stehen,
   - R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

      - (Aʺʺ)-Si(R₆")_{d}"(OR₅")_{c}" (III),

      - c, für eine ganze Zahl von 1 bis 3 steht,
      - d für die ganze Zahl 3 - c steht,
      - c' für eine ganze Zahl von 1 bis 3 steht,
      - d' für die ganze Zahl 3 - c' steht,
      - c" für eine ganze Zahl von 1 bis 3 steht,
      - d" für die ganze Zahl 3 - c" steht,
      - e für 0 oder 1 steht,
      - f für 0 oder 1 steht,
      - g für 0 oder 1 steht,
      - h für 0 oder 1 steht,
      - mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und Aʺʺ in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweibindige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweibindige Alkylengruppen auch verzweigt sein. Beispiele für verzweigte, zweibindige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweibindige C₁-C₂₀-Alkylengruppe steht.

Bevorzugt steht -L- für eine lineare, zweibindige C₁-C₂₀-Alkylengruppe. Weiter bevorzugt steht -L- für eine lineare zweibindige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Der beste Schutz vor den negativen Auswirkungen von Wasser- und/oder Luftverschmutzungen ("Anti-Pollution"-Wirkung) und die beste Pflege von beanspruchtem Haar konnte erhalten werden, wenn das wasserfreie Trägermedium mindestens eine organische Siliciumverbindung der Formel (I) oder Formel (II) enthält, in denen die Reste R₃, R₄ unabhängig voneinander für eine Methylgruppe oder für eine Ethylgruppe stehen.

Besonders gut geeignete organische Siliciumverbindungen der Formel (I) sind
- (3-Aminopropyl)triethoxysilan 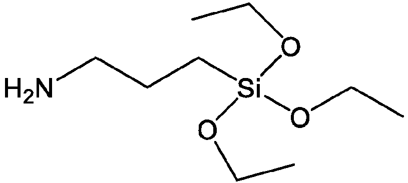
- (3-Aminopropyl)trimethoxysilan 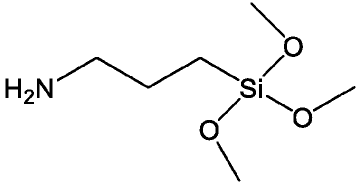
- 1-(3-Aminopropyl)silantriol 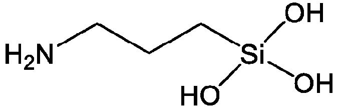
- (2-Aminoethyl)triethoxysilan 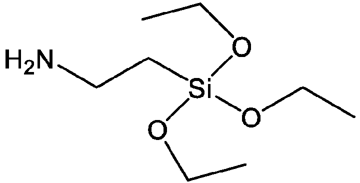
- (2-Aminoethyl)trimethoxysilan 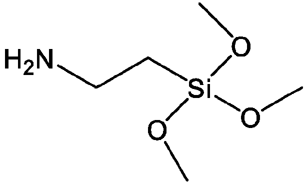
- 1-(2-Aminoethyl)silantriol 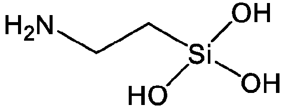
- (3-Dimethylaminopropyl)triethoxysilan 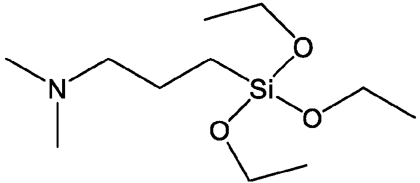
- (3-Dimethylaminopropyl)trimethoxysilan 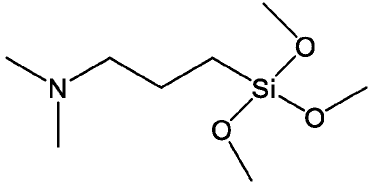
- 1-(3-Dimethylaminopropyl)silantriol 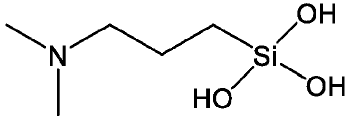
- (2-Dimethylaminoethyl)triethoxysilan. 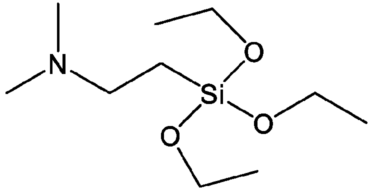
- (2-Dimethylaminoethyl)trimethoxysilan und/oder 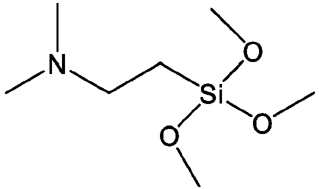
- 1-(2-Dimethylaminoethyl)silantriol

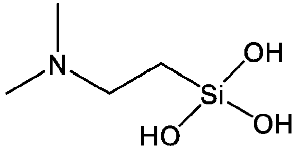

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform enthält das wasserfreie Trägermedium mindestens eine organische Siliciumverbindung der Formel (II)

(R₅O)_{c}(R₆)_{d}Si-(A)ₑ-[NR₇-{A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Sehr bevorzugte Trägermedien enthalten eine organische Siliciumverbindung, bei der die Reste c und c' beide für die Zahl 3 stehen. In diesem Fall stehen d und d' beide für die Zahl 0.

In einer weiteren bevorzugten enthält das wasserfreie Trägermedium mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Pflegewirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (Ilb)

(R₅O)_{c}(R₆)_{d}Si-(A)-[NR₇-(A')]-Si(R₆')_{d'}(OR₅')_{c'} (IIb).

Die Reste A, A', A", A‴ und A"" stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das wasserfreie Trägermedium eine organische Siliciumverbindung mit 3 reaktiven Silan-Gruppen.

In einer weiteren bevorzugten enthält das wasserfreie Trägermedium mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das wasserfreie Trägermedium mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 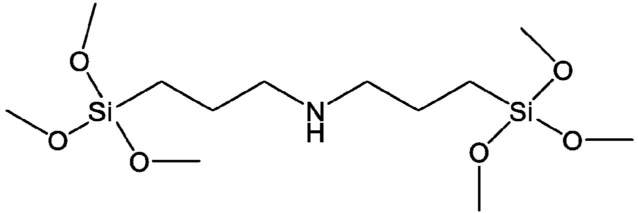
- 3-(Triethoxysilyl)-N-[3-(triethoxysilyl)propyl]-1-propanamin 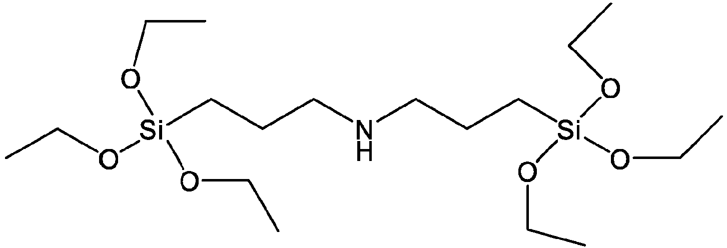
- N-Methyl-3-(trimethoxysilyl)-N-[3-(trimethoxysilyl)propyl]-1-propanamin 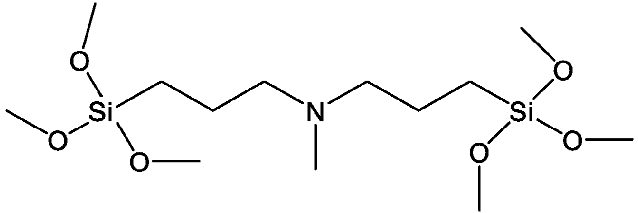
- N-Methyl-3-(triethoxysilyl)-N-[3-(triethoxysilyl)propyl]-1-propanamin 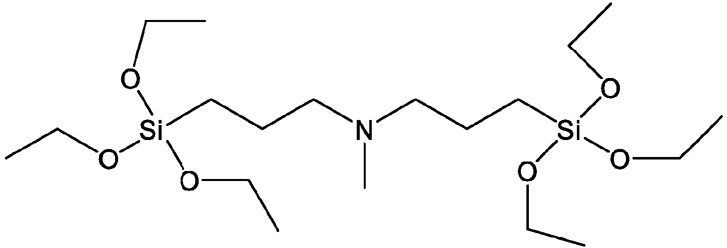
- 2-[Bis[3-(trimethoxysilyl)propyl]amino]-ethanol 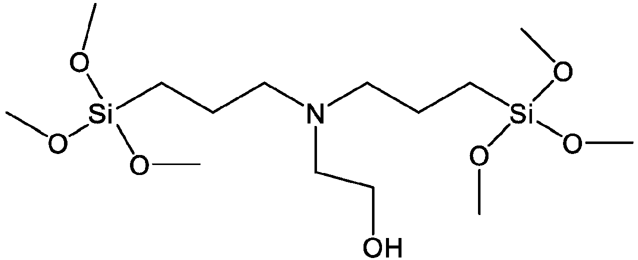
- 2-[Bis[3-(triethoxysilyl)propyl]amino]-ethanol 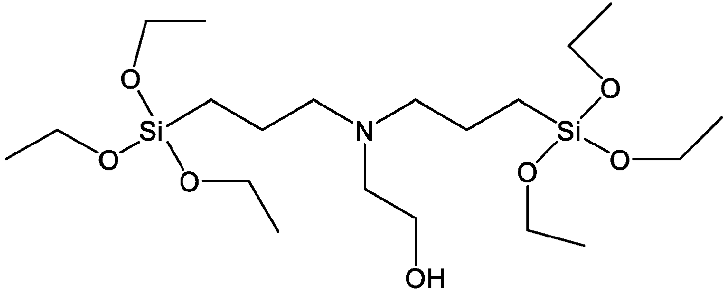
- 3-(Trimethoxysilyl)-N,N-bis[3-(trimethoxysilyl)propyl]-1-propanamin 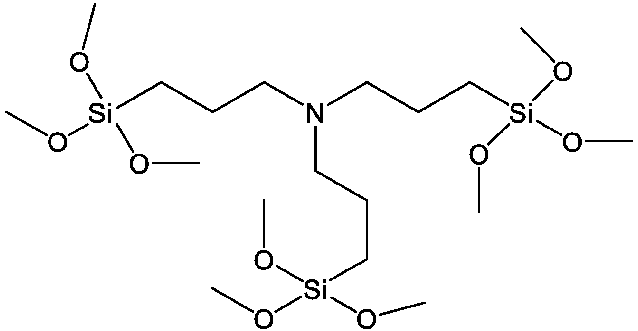
- 3-(Triethoxysilyl)-N,N-bis[3-(triethoxysilyl)propyl]-1-propanamin 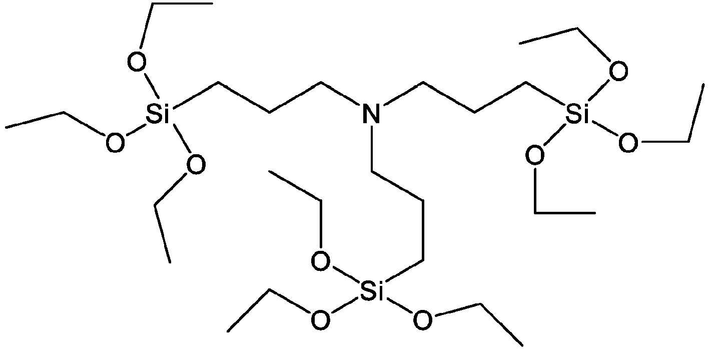
- N1,N1-Bis[3-(trimethoxysilyl)propyl]-1,2-ethanediamin, 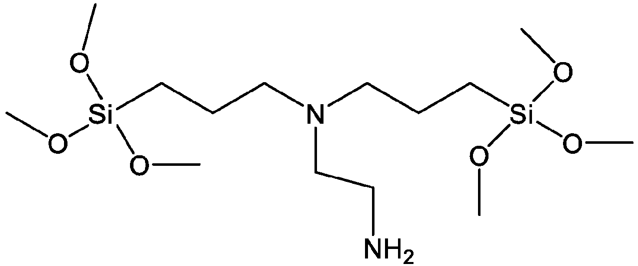
- N1,N1-Bis[3-(triethoxysilyl)propyl]-1,2-ethanediamin, 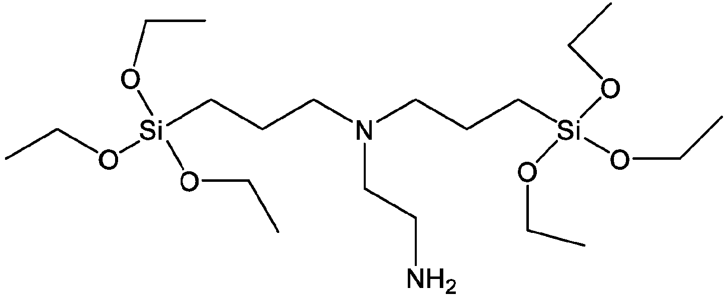
- N,N-Bis[3-(trimethoxysilyl)propyl]-2-propen-1-amin 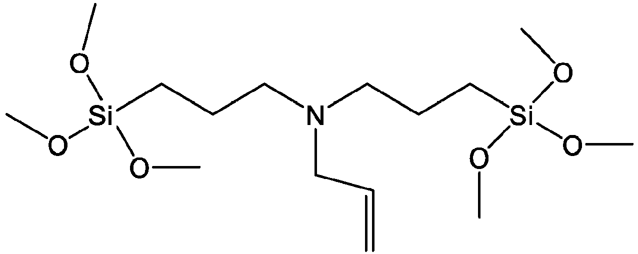
- N,N-Bis[3-(triethoxysilyl)propyl]-2-propen-1-amin

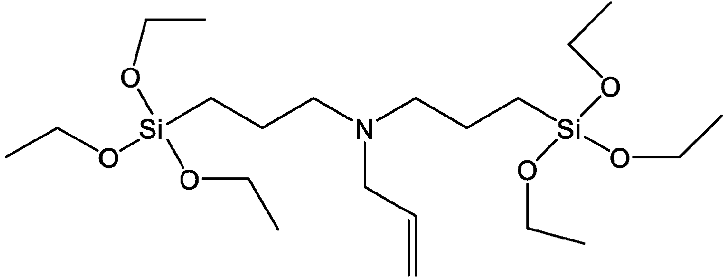

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Es sich ebenfalls als vorteilhaft herausgestellt, wenn das auf dem Haar angewendete Mittel zur Behandlung eines keratinischen Materials mindestens eine organische Siliciumverbindung der Formel (IV) enthält

R₉Si(OR₁₀)ₖ(R₁₁)ₘ (IV).

Somit enthält auch das wasserfreie Trägermedium eine organische Siliciumverbindung der Formel (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform enthält das wasserfreie Trägermedium zusätzlich zu der oder den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer ebenfalls bevorzugten Ausführungsform enthält das wasserfreie Trägermedium zusätzlich zu der oder den organischen Siliciumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform enthält das wasserfreie Trägermedium zusätzlich zu den organischen Siliciumverbindungen der Formel (I) und (II) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe. Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Formel (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Es hat sich als sehr vorteilhaft erwiesen, dass das wasserfreie Trägermedium mindestens eine organische Siliciumverbindung der Formel (IV) enthält, in welcher der Rest k für die Zahl 3 steht. In diesem Fall steht der Rest m für die Zahl 0.

Zur Lösung der Aufgabenstellung besonders gut geeignete organische Siliciumverbindungen der Formel (IV) sind
- Methyltrimethoxysilan 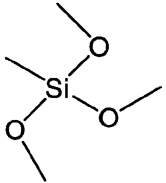
- Methyltriethoxysilan 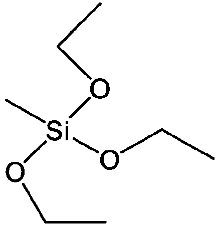
- Ethyltrimethoxysilan 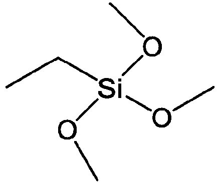
- Ethyltriethoxysilan 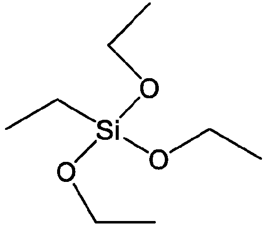
- n-Hexyltrimethoxysilan 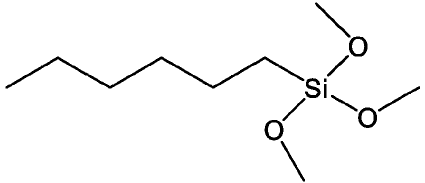
- n-Hexyltriethoxysilan 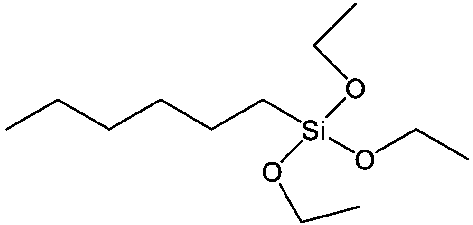
- n-Octyltrimethoxysilan 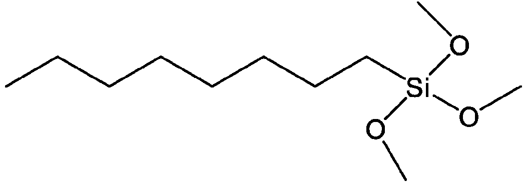
- n-Octyltriethoxysilan 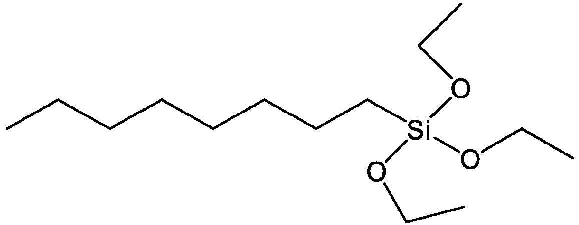
- n-Dodecyltrimethoxysilan und/oder 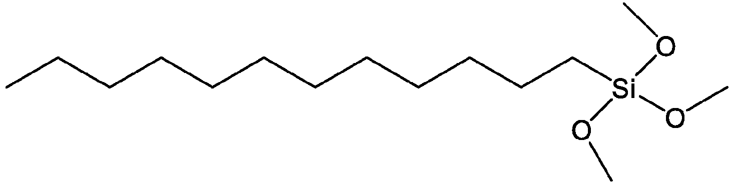
- n-Dodecyltriethoxysilan 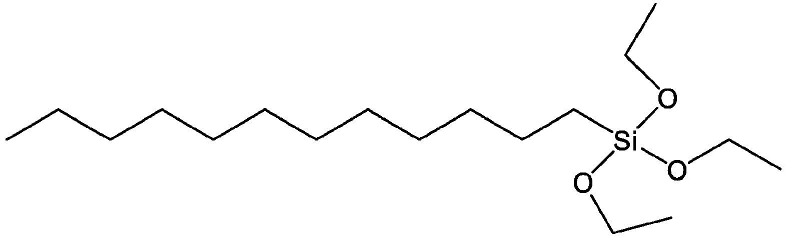
sowie Propyltrimethoxysilan, Propyltriethoxysilan, Octadecyltrimethoxysilan und/oder Octadecyltriethoxysilan.

Bei den zuvor beschriebenen organischen Siliciumverbindungen handelt es sich um reaktive Verbindungen.

Es hat sich herausgestellt, dass auf dem keratinischen Material auch dann besonders stabile und gleichmäßige Filme erhalten werden konnten, wenn das wasserfreie Trägermedium zwei strukturell voneinander verschiedene organische Siliciumverbindungen enthält.

In einer bevorzugten Ausführungsform ist ein wasserfreies Trägermedium dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein wasserfreies Trägermedium dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein wasserfreies Trägermedium dadurch gekennzeichnet, dass das wasserfreie Trägermedium - bezogen auf das Gesamtgewicht des wasserfreien Trägermediums - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem wasserfreien Trägermedium enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem wasserfreien Trägermedium enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des wasserfreien Trägermediums.

Als zweiten erfindungswesentlichen Bestandteil enthält das wasserfreie Trägermedium ein verzweigtes oder lineares Alkan. Im Zuge der zu dieser Erfindung führenden Arbeiten hat sich herausgestellt, dass zur Erzielung einer besonders guten Pflege-Wirkung es insbesondere von Vorteil ist, wenn die organische Siliciumverbindungen, beispielsweise (3-Aminopropyl)triethoxysilan, mit einem verzweigten oder linearen C8-C30 Alkan kombiniert wird. Durch den Ausschluss von Wasser in dem wasserfreien Trägermedium wird die organische Siliciumverbindung vor verfrühter Hydrolyse geschützt und erst bei Bedarf durch Mischen mit einer Wasserphase zu einer Haarpflegeemulsion umgewandelt, welche die organische Siliciumverbindung erst kurz vor der Applikation mittels Hydrolysereaktion aktiviert. Es wurde überraschenderweise gefunden, dass insbesondere die Kombination aus (3-Aminopropyl)triethoxysilan mit C8-C30 Alkanen die kosmetische Akzeptanz steigert. Das Haar ist weich, die Kämmbarkeit deutlich gesteigert und die Haaroberfläche ist besonders bei chemisch behandeltem Haar hydrophober.

Gemäß bevorzugter Ausführungsformen der vorliegenden Erfindung ist das Alkan ein C10-C24 Alkan, bevorzugter ein C12-C18 Alkan und noch bevorzugter ein C14-C16 Alkan. Gemäß einer weiteren bevorzugten Ausführungsform ist das Alkan in einer Menge von 1 bis 50 Gew.-%, bevorzugt 2 bis 45 Gew.-%, bevorzugter 3 bis 40 Gew.-%, noch bevorzugter 4 bis 35 Gew.-%, bezogen auf das Gesamtgewicht des wasserfreien Trägermediums, in dem wasserfreien Trägermedium enthalten.

Die Kombination aus der siliciumorganischen Verbindung, insbesondere (3-Aminopropyl)triethoxysilan, mit dem Alkan bildet eine Schicht auf dem Haar. Die Haaroberfläche wird bei oxidativ geschädigtem Haar wieder hydrophobisiert, was zur Reduktion von Frizz führt. Darüber hinaus wird die Kämmbarkeit des Haares verbessert.

Als dritten erfindungswesentlichen Bestandteil enthält das wasserfreie Trägermedium mindestens einen verzweigten oder linearen C10-C30 Fettalkohol. Mit dieser Formulierung ist stets gemeint, dass die Kohlenwasserstoffkette 10 bis 30 Kohlenstoffatome aufweist.

Gemäß einer bevorzugten Ausführungsform der vorliegenden Erfindung ist der Fettalkohol ein C12-C24 Fettalkohol, bevorzugt ein C14-C18 Fettalkohol. Gemäß einer weiteren bevorzugten Ausführungsform der vorliegenden Erfindung ist der Fettalkohol in einer Menge von 5 bis 50 Gew.-%, bevorzugt 6 bis 45 Gew.-%, bevorzugter 7 bis 40 Gew.-%, noch bevorzugter 8 bis 35 Gew.-%, bezogen auf das Gesamtgewicht des wasserfreien Trägermediums, in dem wasserfreien Trägermedium enthalten.

Als vierten erfindungswesentlichen Bestandteil enthält das wasserfreie Trägermedium mindestens einen verzweigten oder linearen einwertigen C2-C8 Alkohol. Gemäß einer bevorzugten Ausführungsform der vorliegenden Erfindung ist der Alkohol ein C3-C6 Alkohol, bevorzugter ein C4-C5 Alkohol. Gemäß einer weiteren bevorzugten Ausführungsform ist der Alkohol in einer Menge von 4 bis 50 Gew.-%, bevorzugt 6 bis 45 Gew.-%, bevorzugter 8 bis 40 Gew.-%, noch bevorzugter 10 bis 30 Gew.-%, bezogen auf das Gesamtgewicht des wasserfreien Trägermediums, in dem wasserfreien Trägermedium enthalten. Unter einem einwertigen Alkohol ist ein Alkohol mit nur einer OH-Funktion zu verstehen.

Ein zweiter Gegenstand der vorliegenden Erfindung betrifft ein kosmetisches Mittel. Dieses umfasst das wasserfreie Trägermedium gemäß der vorliegenden Erfindung, das mit einer wässrigen Phase oder alternativ auch mit einer weiteren wasserfreien Phase kombiniert wird. Auf diese Weise können kosmetische Mittel bereitgestellt werden, die alle erwünschten Komponenten, die für das kosmetische Mittel vorteilhaft sind, enthalten, wobei die organische Siliciumverbindung vor einer Hydrolyse geschützt ist.

Eine bevorzugte Ausführungsform der vorliegenden Erfindung betrifft ein kosmetisches Mittel, umfassend ein wasserfreies Trägermedium gemäß der vorliegenden Erfindung sowie eine wässrige Phase, wobei das wasserfreie Trägermedium oder die wässrige Phase ein kationisches Tensid umfasst, bevorzugt eine Alkyltrimoniumverbindung mit einer oder mehreren C8-C22, bevorzugter C10-C18, noch bevorzugter C12-C16 Alkylgruppen, oder wobei das kationische Tensid eines der folgenden Formel ist, 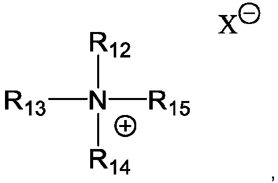 worin
- R₁₂, R₁₃, R₁₄: unabhängig voneinander für eine C1-C6-Alkylgruppe, eine C2-C6-Alkenylgruppe oder eine C2-C6-Hydroxyalkylgruppe stehen,
- R₁₅: für eine C8-C28-Alkylgruppe, bevorzugt eine C10-C22-Alkylgruppe steht und
- X-: für ein physiologisch verträgliches Anion steht,

oder wobei das kationische Tensid eines der folgenden Formel ist, 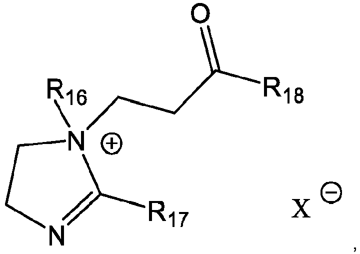 worin
   - R₁₆: für eine C1-C6-Alkylgruppe steht
   - R₁₇, R₁₈: unabhängig voneinander für eine C7-C27-Alkylgruppe, bevorzugt eine C10-C22-Alkylgruppe stehen und
   - X-: für ein physiologisch verträgliches Anion steht,
oder wobei das kationische Tensid eines der folgenden Formel ist, 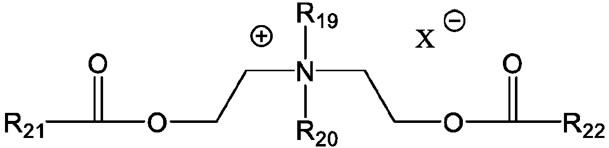 worin
   - R₁₉, R₂₀: unabhängig voneinander für eine C1-C6-Alkylgruppe oder eine C2-C6-Hydroxyalkylgruppe stehen,
   - R₂₁, R₂₂: unabhängig voneinander für eine C7-C27-Alkylgruppe, bevorzugt eine C10-C22-Alkylgruppe stehen und
   - X-: für ein physiologisch verträgliches Anion steht,
oder wobei das kationische Tensid eines der folgenden Formel ist,

   NR₂₃R₂₄R₂₅,

   worin
   - R₂₃, R₂₄: unabhängig voneinander für eine C1-C6-Alkylgruppe, eine C2-C6-Alkenylgruppe oder eine C2-C6-Hydroxyalkylgruppe stehen, und
   - R₂₅: für eine C8-C28-Alkylgruppe, bevorzugt eine C10-C22-Alkylgruppe steht.

Die Tenside dienen in der vorliegenden Erfindung der Emulgierung von wässriger Phase und Trägermedium, bzw. allgemeiner der Emulgierung von Wasser- und Ölphase.

Gemäß einer bevorzugten Ausführungsform der vorliegenden Erfindung ist das kationische Tensid in einer Menge von 0,1 bis 5 Gew.-%, bevorzugt von 0,5 bis 4 Gew.-%, bevorzugter von 1 bis 3 Gew.-%, bezogen auf das Gesamtgewicht der wässrigen Phase, in wässrigen Phase enthalten.

Eine bevorzugte Ausführungsform der vorliegenden Erfindung betrifft ein kosmetisches Mittel, bei dem das wasserfreie Trägermedium oder die wässrige Phase ein nichtionisches Tensid umfasst, wobei das nichtionische Tensid bevorzugt ein Fettalkoholethoxylat ist, bei dem der Fettalkoholteil des Fettalkoholethoxylats eine Alkylkettenlänge von C4-C30, bevorzugt C6-C25, bevorzugter C8-C20, aufweist und/oder bei dem die Anzahl der Ethoxygruppen im Fettalkoholethoxylat bei 2 bis 120, bevorzugt bei 4 bis 100, bevorzugter bei 6 bis 80, noch bevorzugter bei 8 bis 60, am meisten bevorzugt bei 10 bis 40 liegt, und/oder wobei das nichtionische Tensid in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten ist.

Eine weitere bevorzugte Ausführungsform der vorliegenden Erfindung betrifft ein kosmetisches Mittel, bei dem das wasserfreie Trägermedium oder die wässrige Phase ein anionisches Tensid umfasst, wobei das anionische Tensid bevorzugt ein Fettalkoholsulfat mit einer Kettenlänge von C8-C22, bevorzugt von C10-C20, bevorzugter von C12-C18 ist, und/oder wobei das anionische Tensid in einer Menge von 0,1 bis 10 Gew.-%, bevorzugt von 0,25 bis 7,5 Gew.-%, bevorzugter von 0,5 bis 5 Gew.-%, bezogen auf das Gesamtgewicht des wasserfreien Trägermediums, in dem wasserfreien Trägermedium enthalten ist.

Gemäß einer weiteren bevorzugten Ausführungsform der vorliegenden Erfindung liegt das Gewichtsverhältnis von dem wasserfreien Trägermedium zu der wässrigen Phase im Bereich von 1 zu 10 bis 10 zu 1, bevorzugt 5 zu 1 bis 1 zu 5, bevorzugter von 2 zu 1 bis 1 zu 2.

Im Folgenden werden weitere Bestandteile der Haarbehandlungsmittel beschrieben, die neben den zuvor beschriebenen zwingenden Inhaltstoffen in dem wasserfreien Trägermedium oder der wässrigen Phase enthalten sein können.

Gemäß weiterer bevorzugter Ausführungsformen enthält das wasserfreie Trägermedium oder die wässrigen Phase ferner ein Hautbefeuchtungsmittel bzw. weiteres Pflegemittel, das ausgewählt ist aus der Gruppe bestehend aus Glycerin, Harnstoff, Hyaluronsäure, Silanolester der Hyaluronsäure, Panthenol, Taurin, Ceramide, Phytosterole, Aloe Vera Extrakte, Kreatin, Kreatinin, Natriumhyaluronat, Polysaccharide, Biosaccharide gum-1, Gurkenextrakte, Butylenglykol, Propylenglykol, Methylpropandiol, Ethylhexylglycerin, Sorbitol, Aminosäuren, wobei Glycin, Glycin Soja, Histidin, Tyrosin oder Tryptophan besonders bevorzugte Aminosäuren sind, Aminosäurederivate, natürliche Betainverbindungen, Pyrrolidoncarbonsäure oder ein Salz der Pyrrolidoncarbonsäure, Milchsäure, Lactate, insbesondere Natriumlactat, und/oder Ethylhexyloxyglycerin. Insbesondere die Auswahl dieser Hautbefeuchtungsmittel erhöhen den pflegenden Charakter des wasserfreien Trägermediums.

Gemäß einer weiteren bevorzugten Ausführungsform enthält das wasserfreie Trägermedium oder die wässrige Phase mehrere Tenside. Es ist insbesondere bevorzugt, dass das wasserfreie Trägermedium oder die wässrige Phase zwei strukturell voneinander verschiedene Tenside enthält, wobei bevorzugt das wasserfreie Trägermedium oder die wässrige Phase zwei strukturell voneinander verschiedene kationische Tenside, zwei voneinander verschiedene anionische Tenside, ein kationisches Tensid und ein nichtionisches Tensid, oder ein anionisches Tensid und ein nichtionisches Tensid enthält.

Gemäß einer bevorzugten Ausführungsform der vorliegenden Erfindung umfasst das eingesetzte kationische Tensid eine hydrophobe Kopfgruppe mit einer kationischen Ladung und einen oder zwei hydrophobe Endteile, wobei das hydrophobe Endteil oder die hydrophoben Endteile geradkettige oder verzweigte, gesättigte oder ein- oder mehrfach ungesättigte Alkylgruppen darstellen, die bevorzugt eine Kettenlänge von C6 bis C30, bevorzugter C8 bis C26, besonders bevorzugt C10 bis C22, aufweisen. Gemäß einer weiteren bevorzugten Ausführungsform weist das kationische Tensid eine Esterfunktion, eine Etherfunktion, eine Ketonfunktion, eine Alkoholfunktion oder eine Amidfunktion auf.

Bei den kationischen Tensiden der Formel NR₂₃R₂₄R₂₅ handelt es sich um Aminderivate, sogenannte Pseudoquats. Die organischen Reste R₂₃, R₂₄ und R₂₅ sind dabei unmittelbar an das Stickstoffatom gebunden. Im sauren pH-Bereich werden diese kationisiert, d.h. das Stickstoffatom wird dann protoniert. Als Gegenionen bieten sich die physiologisch verträglichen Gegenionen an. Als besonders bevorzugt bietet sich bei diesen kationischen Tensiden Steamidopropyl Dimethylamine an.

Grundsätzlich können als Gegenionen der vorliegenden Komponenten, die als Salze vorliegen, alle physiologisch verträgliche Gegenionen verwendet werden.

Gemäß einer bevorzugten Ausführungsform der vorliegenden Erfindung enthält das wasserfreie Trägermedium oder die wässrige Phase als eine weitere Komponente ein weiteres nichtionisches Tensid, das bevorzugt ein nichtionisches Tensid ausgewählt aus der Gruppe bestehend aus den folgenden umfasst:
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, und
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen.

Gemäß bevorzugter Ausführungsformen der vorliegenden Erfindung sind ein oder mehrere weitere anionische Tenside als eine Komponente in dem wasserfreien Trägermedium oder der wässrigen Phase enthalten, die bevorzugt ausgewählt ist aus der Gruppe bestehend aus
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 16 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12, bevorzugter 2 bis 4 und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist.

Ganz besonders bevorzugt enthält wässrige Phase eine Tensidmischung aus anionischen und amphoteren/zwitterionischen Tensiden Natriumlaurylethersulfat (INCI: Sodium Laureth Sulfate) und ganz besonders bevorzugt Natriumlaurylethersulfat mit 2 Ethylenoxideinheiten.

Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -SO₃⁻ -Gruppe tragen. Unter amphoteren/zwitterionischen Tensiden werden auch solche oberflächenaktiven Verbindungen verstanden, die außer einer C₈ - C₂₄ -Alkyl- oder - Acylgruppe mindestens eine freie Aminogruppe und mindestens eine -COOH- oder -SO₃H-Gruppe enthalten und zur Ausbildung innerer Salze befähigt sind.

Gemäß einer bevorzugten Ausführungsform der vorliegenden Erfindung enthält das wasserfreie Trägermedium oder die wässrige Phase als eine weitere Komponente mindestens ein amphoteres Tensid. Bevorzugt sind die amphoteren Tenside in dem wasserfreie Trägermedium ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten Tenside Cocamidopropylbetain und Disodium Cocoamphodiacetate.

Das wasserfreie Trägermedium zur Stabilisierung von organischen Siliciumverbindungen kann insbesondere verwendet werden zur Herstellung eines Mittels zur Reinigung eines keratinischen Materials, eines Mittels zur Pflege eines keratinischen Materials, eines Mittels zur Pflege und Reinigung eines keratinischen Materials, eines Mittels zur Färbung eines keratinischen Materials und/oder eines Mittels zum temporären Umformen eines keratinischen Materials umfassen.

Es kann bevorzugt sein, dass das wasserfreie Trägermedium oder die wässrige Phase zur Behandlung eines keratinischen Materials ferner 0,001 bis 20 Gew.-% mindestens einer quaternären Verbindung umfasst. Dies gilt insbesondere für wasserfreie Trägermedien oder wässrigen Phasen, die zur Herstellung eines Mittels zur Pflege eines keratinischen Materials oder zur Pflege und Reinigung eines keratinischen Materials verwendet werden.

Es ist bevorzugt, dass die mindestens eine quaternäre Verbindung ausgewählt aus mindestens einer der Gruppen bestehend aus
i) der Monoalkylquats und/oder
ii) der Esterquats und/oder
iii) der quaternären Imidazoline der Formel (Tkat2), 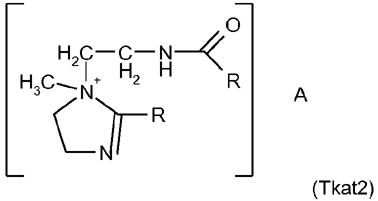 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amidoamine und/oder kationisierten Amidoamine und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationischen Alkylpolyglycosiden und/oder
viii) kationisiertem Honig und/oder
ix) kationischen Guar-Derivaten und/oder
x) Chitosan und/oder
xi) polymeren Dimethyldiallylammoniumsalzen und deren Copolymeren mit Estern und Amiden von Acrylsäure und Methacrylsäure, insbesondere Polyquaternium-7 und/oder
xii) Copolymeren des Vinylpyrrolidons mit quaternierten Derivaten des Dialkylaminoalkylacrylats und -methacrylats, insbesondere Polyquaternium-11 und/oder
xiii) Vinylpyrrolidon-Vinylimidazoliummethochlorid-Copolymeren, insbesondere Polyquaternium-16 und/oder
xiv) quaterniertem Polyvinylalkohol und/oder
xv) Polyquaternium-74,
sowie Mischungen hiervon.

Es ist insbesondere bevorzugt, dass das wasserfreie Trägermedium oder die wässrige Phase ein kationisches Homopolymer, welches unter die INCI-Bezeichnung Polyquaternium-37 fällt, als quaternäre Verbindungen enthält.

Es kann bevorzugt sein, dass das wasserfreie Trägermedium oder die wässrige Phase ferner eine festigende Verbindung, vorzugsweise ausgewählt aus der Gruppe bestehend aus Wachsen, synthetischen Polymeren und Mischungen daraus, umfasst.

Um den unterschiedlichen Anforderungen an kosmetischen Mitteln gerecht zu werden, die zur Herstellung eines Mittels zur Behandlung eines keratinischen Materials zum temporären Umformen eines keratinischen Materials (= Stylingmittel) verwendet werden, sind als festigende Verbindungen bereits eine Vielzahl von synthetischen Polymeren entwickelt worden, die in dem Mittel zur Behandlung eines keratinischen Materials zur Anwendung kommen können. Alternativ oder ergänzend werden Wachse als festigende Verbindungen eingesetzt. Idealerweise ergeben die Polymere und/oder Wachse bei der Anwendung auf dem keratinischen Material einen Polymerfilm oder Film, der einerseits der Frisur einen starken Halt verleiht, andererseits aber hinreichend flexibel ist, um bei Beanspruchung nicht zu brechen.

Die synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VAlCrotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Auch Homopolyacrylsäure (INCI: Carbomer), die im Handel unter dem Namen Carbopol^{®} in unterschiedlichen Ausführungen erhältlich ist, ist als festigende Verbindung geeignet.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Eine ebenfalls bevorzugte festigende Verbindung ist Octylacrylamide/Acrylates/Butylaminoethyl Methacrylate Copolymer (INCI), welches unter der Bezeichnung "Amphomer^{®}" von Akzo Nobel vertrieben wird.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Die kosmetischen Mittel können zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Dazu sollen jedoch keine Alkane gezählt werden, die in dem erfindungsgemäßen wasserfreien Trägermedium zwingend enthalten sind. Es können auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen

Das wasserfreie Trägermedium oder die wässrige Phase enthält die festigende Verbindung vorzugsweise in einer Gesamtmenge von 0,5 bis 50 Gew.-%, bevorzugt 1 bis 40 Gew.-%, weiter bevorzugt 1,5 bis 30 Gew.-%, noch mehr bevorzugt 2 bis 25 Gew.-%, bezogen auf das Gesamtgewicht der kosmetischen Zusammensetzung.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflanzenextrakte, Silikone, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den bevorzugten Ausführungsformen 1 bis 48 werden die bevorzugt verwendeten organischen Siliciumverbindungen mit den bevorzugt verwendeten Alkanen und Alkoholen in einem erfindungsgemäßen wasserfreiem Trägermedium miteinander kombiniert.

| | Silanverbindung | weiterere Inhaltsstoffe |
|---|---|---|
| 1 | (3-Aminopropyl)trimethoxysilan | verzweigtes oder lineares C14-C16 Alkan + verzweigter oder linearer C14-C18 Fettalkohol + verzweigter oder linearer einwertiger C4-C5 Alkohol |
| 2 | (3-Aminopropyl)triethoxysilan | verzweigtes oder lineares C14-C16 Alkan + verzweigter oder linearer C14-C18 Fettalkohol + verzweigter oder linearer einwertiger C4-C5 Alkohol |
| 3 | (2-Aminoethyl)trimethoxysilan | verzweigtes oder lineares C14-C16 Alkan + verzweigter oder linearer C14-C18 Fettalkohol + verzweigter oder linearer einwertiger C4-C5 Alkohol |
| 4 | (2-Aminoethyl)triethoxysilan | verzweigtes oder lineares C14-C16 Alkan + verzweigter oder linearer C14-C18 Fettalkohol + verzweigter oder linearer einwertiger C4-C5 Alkohol |
| 5 | (3-Dimethylaminopropyl)trimethoxysilan | verzweigtes oder lineares C14-C16 Alkan + verzweigter oder linearer C14-C18 Fettalkohol + verzweigter oder linearer einwertiger C4-C5 Alkohol |
| 6 | (3-Dimethylaminopropyl)triethoxysilan | verzweigtes oder lineares C14-C16 Alkan + verzweigter oder linearer C14-C18 Fettalkohol + verzweigter oder linearer einwertiger C4-C5 Alkohol |
| 7 | (2-Dimethylaminoethyl)trimethoxysilan | verzweigtes oder lineares C14-C16 Alkan + verzweigter oder linearer C14-C18 Fettalkohol + verzweigter oder linearer einwertiger C4-C5 Alkohol |
| 8 | (2-Dimethylaminoethyl)triethoxysilan | verzweigtes oder lineares C14-C16 Alkan + verzweigter oder linearer C14-C18 Fettalkohol + verzweigter oder linearer einwertiger C4-C5 Alkohol |
| 9 | 3-(Trimethoxysilyl)-N-[3-(trimethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 10 | 3-(Triethoxysilyl)-N-[3-(triethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 11 | N-Methyl-3-(trimethoxysilyl)-N-[3-(trimethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 12 | N-Methyl-3-(triethoxysilyl)-N-[3-(triethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 13 | 2-[Bis[3-(trimethoxysilyl)propyl]amino]-ethanol | verzweigtes oder lineares C14-C16 Alkan + verzweigter oder linearer C14-C18 Fettalkohol + verzweigter oder linearer einwertiger C4-C5 Alkohol |
| 14 | 2-[Bis[3-(triethoxysilyl)propyl]amino]-ethanol | verzweigtes oder lineares C14-C16 Alkan + verzweigter oder linearer C14-C18 Fettalkohol + verzweigter oder linearer einwertiger C4-C5 Alkohol |
| 15 | 3-(Trimethoxysilyl)-N,N-bis[3-(trimethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 16 | 3-(Triethoxysilyl)-N,N-bis[3-(triethoxysilyl)propyl]-1-propanamin | verzweigtes oder lineares C14-C16 Alkan + verzweigter oder linearer C14-C18 Fettalkohol + verzweigter oder linearer einwertiger C4-C5 Alkohol |
| 17 | N1,N1-Bis[3-(trimethoxysilyl)propyl]-1,2-ethandiamin | verzweigtes oder lineares C14-C16 Alkan + verzweigter oder linearer C14-C18 Fettalkohol + verzweigter oder linearer einwertiger C4-C5 Alkohol |
| 18 | N1,N 1-Bis[3-(triethoxysilyl)propyl]-1,2-ethandiamin | verzweigtes oder lineares C14-C16 Alkan + verzweigter oder linearer C14-C18 Fettalkohol + verzweigter oder linearer einwertiger C4-C5 Alkohol |
| 19 | N,N-Bis[3-(trimethoxysilyl)propyl]-2-propen-1-amin | verzweigtes oder lineares C14-C16 Alkan + verzweigter oder linearer C14-C18 Fettalkohol + verzweigter oder linearer einwertiger C4-C5 Alkohol |
| 20 | N,N-Bis[3-(triethoxysilyl)propyl]-2-propen-1-amin | verzweigtes oder lineares C14-C16 Alkan + verzweigter oder linearer C14-C18 Fettalkohol + verzweigter oder linearer einwertiger C4-C5 Alkohol |
| 21 | Methyltrimethoxysilan | verzweigtes oder lineares C14-C16 Alkan + verzweigter oder linearer C14-C18 Fettalkohol + verzweigter oder linearer einwertiger C4-C5 Alkohol |
| 22 | Methyltriethoxysilan | verzweigtes oder lineares C14-C16 Alkan + verzweigter oder linearer C14-C18 Fettalkohol + verzweigter oder linearer einwertiger C4-C5 Alkohol |
| 23 | Ethyltrimethoxysilan | verzweigtes oder lineares C14-C16 Alkan + verzweigter oder linearer C14-C18 Fettalkohol + verzweigter oder linearer einwertiger C4-C5 Alkohol |
| 24 | Ethyltriethoxysilan | verzweigtes oder lineares C14-C16 Alkan + verzweigter oder linearer C14-C18 Fettalkohol + verzweigter oder linearer einwertiger C4-C5 Alkohol |
| 25 | Propyltrimethoxysilan | verzweigtes oder lineares C14-C16 Alkan + verzweigter oder linearer C14-C18 Fettalkohol + verzweigter oder linearer einwertiger C4-C5 Alkohol |
| 26 | Propyltriethoxysilan | verzweigtes oder lineares C14-C16 Alkan + verzweigter oder linearer C14-C18 Fettalkohol + verzweigter oder linearer einwertiger C4-C5 Alkohol |
| 27 | Hexyltrimethoxysilan | verzweigtes oder lineares C14-C16 Alkan + verzweigter oder linearer C14-C18 Fettalkohol + verzweigter oder linearer einwertiger C4-C5 Alkohol |
| 28 | Hexyltriethoxysilan | verzweigtes oder lineares C14-C16 Alkan + verzweigter oder linearer C14-C18 Fettalkohol + verzweigter oder linearer einwertiger C4-C5 Alkohol |
| 29 | Octyltrimethoxysilan | verzweigtes oder lineares C14-C16 Alkan + verzweigter oder linearer C14-C18 Fettalkohol + verzweigter oder linearer einwertiger C4-C5 Alkohol |
| 30 | Octyltriethoxysilan | verzweigtes oder lineares C14-C16 Alkan + verzweigter oder linearer C14-C18 Fettalkohol + verzweigter oder linearer einwertiger C4-C5 Alkohol |
| 31 | Dodecyltrimethoxysilan | verzweigtes oder lineares C14-C16 Alkan + verzweigter oder linearer C14-C18 Fettalkohol + verzweigter oder linearer einwertiger C4-C5 Alkohol |
| 32 | Dodecyltriethoxysilan | verzweigtes oder lineares C14-C16 Alkan + verzweigter oder linearer C14-C18 Fettalkohol + verzweigter oder linearer einwertiger C4-C5 Alkohol |
| 33 | Octadecyltrimethoxysilan | verzweigtes oder lineares C14-C16 Alkan + verzweigter oder linearer C14-C18 Fettalkohol + verzweigter oder linearer einwertiger C4-C5 Alkohol |
| 34 | Octadecyltriethoxysilan | verzweigtes oder lineares C14-C16 Alkan + verzweigter oder linearer C14-C18 Fettalkohol + verzweigter oder linearer einwertiger C4-C5 Alkohol |
| 35 | (3-Aminopropyl)triethoxysilan + Methyltrimethoxysilan | verzweigtes oder lineares C14-C16 Alkan + verzweigter oder linearer C14-C18 Fettalkohol + verzweigter oder linearer einwertiger C4-C5 Alkohol |
| 36 | (3-Aminopropyl)triethoxysilan + Methyltriethoxysilan | verzweigtes oder lineares C14-C16 Alkan + verzweigter oder linearer C14-C18 Fettalkohol + verzweigter oder linearer einwertiger C4-C5 Alkohol |
| 37 | (3-Aminopropyl)triethoxysilan + Ethyltrimethoxysilan | verzweigtes oder lineares C14-C16 Alkan + verzweigter oder linearer C14-C18 Fettalkohol + verzweigter oder linearer einwertiger C4-C5 Alkohol |
| 38 | (3-Aminopropyl)triethoxysilan + Ethyltriethoxysilan | verzweigtes oder lineares C14-C16 Alkan + verzweigter oder linearer C14-C18 Fettalkohol + verzweigter oder linearer einwertiger C4-C5 Alkohol |
| 39 | (3-Aminopropyl)triethoxysilan + Propyltrimethoxysilan | verzweigtes oder lineares C14-C16 Alkan + verzweigter oder linearer C14-C18 Fettalkohol + verzweigter oder linearer einwertiger C4-C5 Alkohol |
| 40 | (3-Aminopropyl)triethoxysilan + Propyltriethoxysilan | verzweigtes oder lineares C14-C16 Alkan + verzweigter oder linearer C14-C18 Fettalkohol + verzweigter oder linearer einwertiger C4-C5 Alkohol |
| 41 | (3-Aminopropyl)triethoxysilan + Hexyltrimethoxysilan | verzweigtes oder lineares C14-C16 Alkan + verzweigter oder linearer C14-C18 Fettalkohol + verzweigter oder linearer einwertiger C4-C5 Alkohol |
| 42 | (3-Aminopropyl)triethoxysilan + Hexyltriethoxysilan | verzweigtes oder lineares C14-C16 Alkan + verzweigter oder linearer C14-C18 Fettalkohol + verzweigter oder linearer einwertiger C4-C5 Alkohol |
| 43 | (3-Aminopropyl)triethoxysilan + Octyltrimethoxysilan | verzweigtes oder lineares C14-C16 Alkan + verzweigter oder linearer C14-C18 Fettalkohol + verzweigter oder linearer einwertiger C4-C5 Alkohol |
| 44 | (3-Aminopropyl)triethoxysilan + Octyltriethoxysilan | verzweigtes oder lineares C14-C16 Alkan + verzweigter oder linearer C14-C18 Fettalkohol + verzweigter oder linearer einwertiger C4-C5 Alkohol |
| 45 | (3-Aminopropyl)triethoxysilan + Dodecyltrimethoxysilan | verzweigtes oder lineares C14-C16 Alkan + verzweigter oder linearer C14-C18 Fettalkohol + verzweigter oder linearer einwertiger C4-C5 Alkohol |
| 46 | (3-Aminopropyl)triethoxysilan + Dodecyltriethoxysilan | verzweigtes oder lineares C14-C16 Alkan + verzweigter oder linearer C14-C18 Fettalkohol + verzweigter oder linearer einwertiger C4-C5 Alkohol |
| 47 | (3-Aminopropyl)triethoxysilan + Octadecyltrimethoxysilan | verzweigtes oder lineares C14-C16 Alkan + verzweigter oder linearer C14-C18 Fettalkohol + verzweigter oder linearer einwertiger C4-C5 Alkohol |
| 48 | (3-Aminopropyl)triethoxysilan + Octadecyltriethoxysilan | verzweigtes oder lineares C14-C16 Alkan + verzweigter oder linearer C14-C18 Fettalkohol + verzweigter oder linearer einwertiger C4-C5 Alkohol |

Ein weiterer Gegenstand der vorliegenden Anmeldung ist die Verwendung des erfindungsgemäßen wasserfreien Trägermediums zur Erhöhung der Lagerstabilität von organischen Siliciumverbindungen und/oder zur Herstellung eines Mittels zur Reinigung eines keratinischen Materials, eines Mittels zur Pflege eines keratinischen Materials, eines Mittels zur Pflege und Reinigung eines keratinischen Materials, eines Mittels zur Färbung eines keratinischen Materials und/oder eines Mittels zum temporären Umformen eines keratinischen Materials.

Bezüglich weiterer bevorzugter Ausführungsformen der Verwendung gilt mutatis mutandis das zu den wasserfreien Trägermedien Gesagte.

## Patentansprüche

1. Wasserfreies Trägermedium zur Lagerstabilisierung von organischen Siliciumverbindungen, umfassend
a) mindestens eine organische Siliciumverbindung, wobei die mindestens eine organische Siliciumverbindung eine Verbindung der Formel (I) und mindestens eine organische Siliciumverbindung der Formel (IV) enthält,
wobei in der organischen Siliciumverbindung der Formel (I)
R₁R_{z}N-L-Si(OR₃)ₐ(R₄)_{b} (I),
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht, und
wobei in der organischen Siliciumverbindung der Formel (IV)
R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,
b) mindestens ein verzweigtes oder lineares C8-C30 Alkan,
c) mindestens einen verzweigten oder linearen C10-C30 Fettalkohol, und
d) mindestens einen verzweigten oder linearen einwertigen C2-C8 Alkohol.

2. Wasserfreies Trägermedium zur Lagerstabilisierung gemäß Anspruch 1,
**dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (Aʺʺ)-Si(R₆")_{d"}(OR_{5"})_{c"} (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Wasserfreies Trägermedium zur Lagerstabilisierung gemäß Anspruch 2,
**dadurch gekennzeichnet,**
**dass** das wasserfreie Trägermedium mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und
- N,N-Bis[3-(triethoxysilyl)propyl]-2-Propen-1-amin.

4. Wasserfreies Trägermedium zur Lagerstabilisierung gemäß einem der Ansprüche 1 bis 3, **dadurch gekennzeichnet, dass** das wasserfreie Trägermedium zur Lagerstabilisierung mindestens eine organische Siliciumverbindung der Formel (IV) enthält,
R₉Si(OR₁₀)ₖ(R₁₁)ₘ (IV),
die ausgewählt ist aus der Gruppe bestehend aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan
- Dodecyltriethoxysilan
- Octadecyltrimethoxysilan und
- Octadecyltriethoxysilan.

5. Wasserfreies Trägermedium zur Lagerstabilisierung gemäß einem der Ansprüche 1 bis 4, **dadurch gekennzeichnet, dass** die organische Siliciumverbindung in einer Menge von 0,01 bis 10 Gew.-%, bevorzugt von 0,02 bis 9 Gew.-%, bevorzugter von 0,05 bis 8 Gew.-%, am meisten bevorzugt von 0,1 bis 7 Gew.-%, bezogen auf das Gesamtgewicht des wasserfreien Trägermediums, in dem wasserfreien Trägermedium enthalten ist.

6. Wasserfreies Trägermedium zur Lagerstabilisierung gemäß einem der Ansprüche 1 bis 5, **dadurch gekennzeichnet, dass** das Alkan ein C10-C24 Alkan, bevorzugt ein C12-C18 Alkan und bevorzugter ein C14-C16 Alkan ist.

7. Kosmetisches Mittel, umfassend ein wasserfreies Trägermedium gemäß einem der Ansprüche 1 bis 6 sowie eine wässrige Phase, **dadurch gekennzeichnet, dass** das wasserfreie Trägermedium oder die wässrige Phase ein kationisches Tensid umfasst, bevorzugt eine Alkyltrimoniumverbindung mit einer oder mehreren C8-C22, bevorzugter C10-C18, noch bevorzugter C12-C16 Alkylgruppen, oder wobei das kationische Tensid eines der folgenden Formel ist, 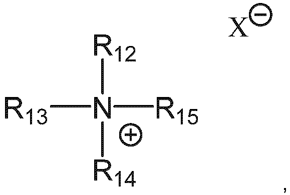 worin
R₁₂, R₁₃, R₁₄ unabhängig voneinander für eine C1-C6-Alkylgruppe, eine C2-C6-Alkenylgruppe oder eine C2-C6-Hydroxyalkylgruppe stehen,
R₁₅ für eine C8-C28-Alkylgruppe, bevorzugt eine C10-C22-Alkylgruppe steht und
X- für ein physiologisch verträgliches Anion steht,
oder wobei das kationische Tensid eines der folgenden Formel ist, 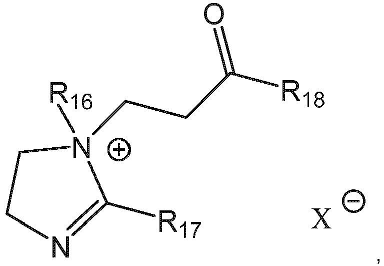 worin
R₁₆ für eine C1-C6-Alkylgruppe steht
R₁₇, R₁₈ unabhängig voneinander für eine C7-C27-Alkylgruppe, bevorzugt eine C10-C22-Alkylgruppe stehen und
X- für ein physiologisch verträgliches Anion steht,
oder wobei das kationische Tensid eines der folgenden Formel ist, 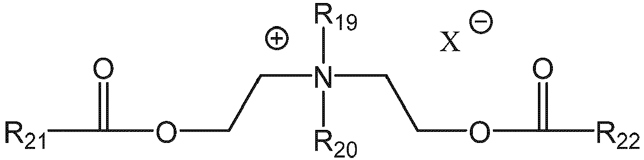 worin
R₁₉, R₂₀ unabhängig voneinander für eine C1-C6-Alkylgruppe oder eine C2-C6-Hydroxyalkylgruppe stehen,
R₂₁, R₂₂ unabhängig voneinander für eine C7-C27-Alkylgruppe, bevorzugt eine C10-C22-Alkylgruppe stehen und
X- für ein physiologisch verträgliches Anion steht,
oder wobei das kationische Tensid eines der folgenden Formel ist,
NR₂₃R₂₄R₂₅,
worin
R₂₃, R₂₄ unabhängig voneinander für eine C1-C6-Alkylgruppe, eine C2-C6-Alkenylgruppe oder eine C2-C6-Hydroxyalkylgruppe stehen, und
R₂₅ für eine C8-C28-Alkylgruppe, bevorzugt eine C10-C22-Alkylgruppe steht, und/oder
**dadurch gekennzeichnet, dass** das kationische Tensid in einer Menge von 0,1 bis 5 Gew.-%, bevorzugt von 0,5 bis 4 Gew.-%, bevorzugter von 1 bis 3 Gew.-%, bezogen auf das Gesamtgewicht der wässrigen Phase, in der wässrigen Phase enthalten ist.

8. Kosmetisches Mittel nach Anspruch 7, **dadurch gekennzeichnet, dass** das wasserfreie Trägermedium oder die wässrige Phase ein nichtionisches Tensid umfasst, wobei das nichtionische Tensid bevorzugt ein Fettalkoholethoxylat ist, bei dem der Fettalkoholteil des Fettalkoholethoxylats eine Alkylkettenlänge von C4-C30, bevorzugt C6-C25, bevorzugter C8-C20, aufweist und/oder bei dem die Anzahl der Ethoxygruppen im Fettalkoholethoxylat bei 2 bis 120, bevorzugt bei 4 bis 100, bevorzugter bei 6 bis 80, noch bevorzugter bei 8 bis 60, am meisten bevorzugt bei 10 bis 40 liegt.

9. Kosmetisches Mittel nach Anspruch 7 oder Anspruch 8, **dadurch gekennzeichnet, dass** das wasserfreie Trägermedium oder die wässrige Phase, ein anionisches Tensid umfasst, wobei das anionische Tensid bevorzugt ein Fettalkoholsulfat mit einer Kettenlänge von C8-C22, bevorzugt von C10-C20, bevorzugter von C12-C18 ist.

10. Kosmetisches Mittel nach einem der Ansprüche 7 bis 9, **dadurch gekennzeichnet, dass** das Gewichtsverhältnis von dem wasserfreien Trägermedium zu der wässrigen Phase im Bereich von 1 zu 10 bis 10 zu 1, bevorzugt 5 zu 1 bis 1 zu 5, bevorzugter von 2 zu 1 bis 1 zu 2 liegt.

## Claims

1. An anhydrous carrier medium for storage stabilization of organosilicon compounds, the medium comprising
a) at least one organosilicon compound, wherein the at least one organosilicon compound contains a compound of formula (I) and at least one organosilicon compound of formula (IV),
where, in the organosilicon compound of formula (I),
R₁R₂N-L-Si(OR₃)ₐ(R₄)_{b} (I),
- R₁ and R₂ both represent a hydrogen atom,
- L represents a linear, divalent C₁-C₆ alkylene group, preferably a propylene group (-CH₂-CH₂-CH₂-) or an ethylene group (-CH₂-CH₂-),
- R₃ and R₄ independently represent a methyl group or an ethyl group,
- a represents the number 3, and
- b represents the number 0, and
where, in the organosilicon compound of formula (IV),
R₉Si(OR₁₀)ₖ(R₁₁)ₘ (IV),
- R₉ represents a C₁-C₁₂ alkyl group,
- R₁₀ represents a hydrogen atom or a C₁-C₆ alkyl group,
- R₁₁ represents a C₁-C₆ alkyl group, and
- k represents an integer from 1 to 3, and
- m represents the integer 3 - k,
b) at least one branched or linear C8-C30 alkane,
c) at least one branched or linear C10-C30 fatty alcohol, and
d) at least one branched or linear monohydric C2-C8 alcohol.

2. The anhydrous carrier medium for storage stabilization according to claim 1,
**characterized in that**
the at least one organosilicon compound further contains a compound of formula (II),
where, in the organosilicon compound of formula (II),
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} and R_{6"} independently represent a C₁-C₆ alkyl group,
- A, A', A", A‴ and Aʺʺ independently represent a linear or branched, divalent C₁-C₂₀ alkylene group,
- R₇ and R₈ independently represent a hydrogen atom, a C₁-C₆-alkyl group, a hydroxy C₁-C₆-alkyl group, a C₂-C₆-alkenyl group, an amino C₁-C₆-alkyl group, or a grouping of formula (III),
- (Aʺʺ)-Si(R₆")_{d"}(OR₅")_{c"} (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The anhydrous carrier medium for storage stabilization according to claim 2,
**characterized in that**
the anhydrous carrier medium contains at least one organosilicon compound of formula (II) which is selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-methyl-3-(triethoxysilyl)-N-[3-(triethoxysilyl)propyl]-1-propanamine
- 2-[bis[3-(trimethoxysilyl)propyl]amino]ethanol
- 2-[bis[3-(triethoxysilyl)propyl]amino]ethanol
- 3-(trimethoxysilyl)-N,N-bis[3-(trimethoxysilyl)propyl]-1-propanamine
- 3-(triethoxysilyl)-N,N-bis[3-(triethoxysilyl)propyl]-1-propanamine
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(trimethoxysilyl)propyl]-2-propen-1-amine, and
- N,N-bis[3-(triethoxysilyl)propyl]-2-propen-1-amine.

4. The anhydrous carrier medium for storage stabilization according to one of claims 1 to 3, **characterized in that** the anhydrous carrier medium for storage stabilization contains at least one organosilicon compound of formula (IV),
R₉Si(OR₁₀)ₖ(R₁₁)ₘ (IV),
which is selected from the group consisting of
- methyltrimethoxysilane
- methyltriethoxysilane
- ethyltrimethoxysilane
- ethyltriethoxysilane
- propyltrimethoxysilane
- propyltriethoxysilane
- hexyltrimethoxysilane
- hexyltriethoxysilane
- octyltrimethoxysilane
- octyltriethoxysilane
- dodecyltrimethoxysilane
- dodecyltriethoxysilane
- octadecyltrimethoxysilane and
- octadecyltriethoxysilane.

5. The anhydrous carrier medium for storage stabilization according to one of claims 1 to 4, **characterized in that** the organosilicon compound is contained in the anhydrous carrier medium in an amount of from 0.01 to 10 wt.%, preferably from 0.02 to 9 wt.%, more preferably from 0.05 to 8 wt.%, most preferably from 0.1 to 7 wt.%, based on the total weight of the anhydrous carrier medium.

6. The anhydrous carrier medium for storage stabilization according to one of claims 1 to 5, **characterized in that** the alkane is a C10-C24 alkane, preferably a C12-C18 alkane and more preferably a C14-C16 alkane.

7. A cosmetic agent comprising an anhydrous carrier medium according to one of claims 1 to 6 and an aqueous phase, **characterized in that** the anhydrous carrier medium or the aqueous phase comprises a cationic surfactant, preferably an alkyltrimonium compound having one or more C8-C22, more preferably C10-C18, even more preferably C12-C16 alkyl groups, or the cationic surfactant being one of the following formula, 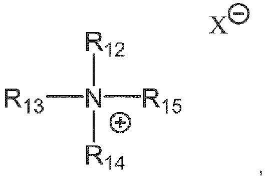 where
R₁₂, R₁₃ and R₁₄ independently represent a C1-C6 alkyl group, a C2-C6 alkenyl group or a C2-C6 hydroxyalkyl group,
R₁₅ represents a C8-C28 alkyl group, preferably a C10-C22 alkyl group and
X- represents a physiologically acceptable anion,
or the cationic surfactant being one of the following formula, 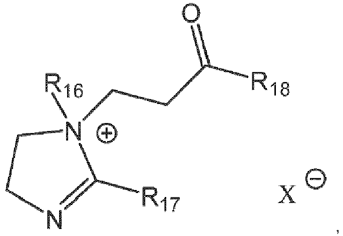 where
R₁₆ represents a C1-C6 alkyl group,
R₁₇ and R₁₈ independently represent a C7-C27 alkyl group, preferably a C10-C22 alkyl group and
X- represents a physiologically acceptable anion,
or the cationic surfactant being one of the following formula, 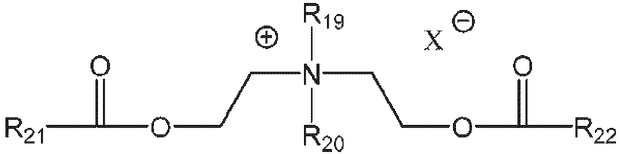 where
R₁₉ and R₂₀ independently represent a C1-C6 alkyl group or a C2-C6 hydroxyalkyl group,
R₂₁ and R₂₂ independently represent a C7-C27 alkyl group, preferably a C10-C22 alkyl group and
X- represents a physiologically acceptable anion,
or the cationic surfactant being one of the following formula,
NR₂₃R₂₄R₂₅,
where
R₂₃ and R₂₄ independently represent a C1-C6 alkyl group, a C2-C6 alkenyl group or a C2-C6 hydroxyalkyl group, and
R₂₅ represents a C8-C28 alkyl group, preferably a C10-C22 alkyl group, and/or
**characterized in that** the cationic surfactant is contained in the aqueous phase in an amount of 0.1 to 5 wt.%, preferably 0.5 to 4 wt.%, more preferably 1 to 3 wt.%, based on the total weight of the aqueous phase.

8. The cosmetic agent according to claim 7, **characterized in that** the anhydrous carrier medium or the aqueous phase comprises a nonionic surfactant, the nonionic surfactant being preferably a fatty alcohol ethoxylate in which the fatty alcohol part of the fatty alcohol ethoxylate has an alkyl chain length of C4-C30, preferably C6-C25, more preferably C8-C20, and/or in which the number of ethoxy groups in the fatty alcohol ethoxylate is 2 to 120, preferably 4 to 100, more preferably 6 to 80, even more preferably 8 to 60, most preferably 10 to 40.

9. The cosmetic agent according to claim 7 or claim 8, **characterized in that** the anhydrous carrier medium or the aqueous phase comprises an anionic surfactant, the anionic surfactant being preferably a fatty alcohol sulfate having a chain length of C8-C22, preferably C10-C20, more preferably C12-C18.

10. The cosmetic agent according to one of claims 7 to 9,
**characterized in that** the weight ratio of the anhydrous carrier medium to the aqueous phase is in the range from 1 to 10 to 10 to 1, preferably 5 to 1 to 1 to 5, more preferably from 2 to 1 to 1 to 2.

## Revendications

1. Milieu de support anhydre pour la stabilité au stockage de composés organiques de silicium, comprenant
a) au moins un composé organique de silicium, dans lequel l'au moins un composé organique de silicium contient un composé de formule (I) et au moins un composé organique de silicium de formule (IV),
où, dans le composé organique de silicium de formule (I)
R₁R₂N-L-Si(OR₃)ₐ(R₄)_{b} (I),
- R₁, R₂ représentent tous deux un atome d'hydrogène,
- L représente un groupe alkylène en C₁-C₆ bivalent linéaire, de préférence un groupe propylène (-CH₂-CH₂-CH₂-) ou un groupe éthylène (-CH₂-CH₂-),
- R₃, R₄ représentent, indépendamment l'un de l'autre, un groupe méthyle ou un groupe éthyle,
- a représente le nombre 3 et
- b représente le nombre 0, et
où, dans le composé organique de silicium de formule (IV)
R₉Si(OR₁₀)ₖ(R₁₁)ₘ (IV),
- R₉ représente un groupe alkyle en C₁-C₁₂,
- R₁₀ représente un atome d'hydrogène ou un groupe alkyle en C₁-C₆,
- R₁₁ représente un groupe alkyle en C₁-C₆
- k représente un nombre entier allant de 1 à 3, et
- m représente le nombre entier 3 - k,
b) au moins un alcane en C8-C30 ramifié ou linéaire,
c) au moins un alcool gras en C10-C30 ramifié ou linéaire, et
d) au moins un alcool en C2-C8 monovalent, ramifié ou linéaire.

2. Milieu de support anhydre pour la stabilité au stockage selon la revendication 1,
**caractérisé en ce que**
l'au moins un composé organique de silicium contient en outre un composé de formule (II),
où, dans le composé organique de silicium de formule (II)
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} et R_{6"} représentent, indépendamment les uns des autres, un groupe alkyle en C₁-C₆,
- A, A', A", A‴ et Aʺʺ représentent, indépendamment les uns des autres, un groupe alkylène en C₁-C₂₀ bivalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
- (Aʺʺ)-Si(R₆")_{d"}(OR₅")_{c"} (III),
- c représente un nombre entier allant de 1 à 3,
- d représente le nombre entier 3 - c,
- c' représente un nombre entier allant de 1 à 3,
- d' représente le nombre entier 3 - c',
- c" représente un nombre entier allant de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Milieu de support anhydre pour la stabilité au stockage selon la revendication 2,
**caractérisé en ce que**
le milieu de support anhydre contient au moins un composé organique de silicium de formule (II) qui est choisi dans le groupe constitué de
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propén-1-amine et
- N,N-bis[3-(triéthoxysilyl)propyl]-2-propén-1-amine.

4. Milieu de support anhydre pour la stabilité au stockage selon l'une des revendications 1 à 3, **caractérisé en ce que** le milieu de support anhydre pour la stabilité au stockage contient au moins un composé organique de silicium de formule (IV),
R₉Si(OR₁₀)ₖ(R₁₁)ₘ (IV),
lequel est choisi dans le groupe constitué de
- méthyltriméthoxysilane
- méthyltriéthoxysilane
- éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane
- dodécyltriéthoxysilane
- octadécyltriméthoxysilane et
- octadécyltriéthoxysilane.

5. Milieu de support anhydre pour la stabilité au stockage selon l'une des revendications 1 à 4, **caractérisé en ce que** le composé organique de silicium est contenu dans le milieu de support anhydre en une quantité allant de 0,01 à 10 % en poids, de préférence de 0,02 à 9 % en poids, plus préférablement de 0,05 à 8 % en poids, le plus préférablement de 0,1 à 7 % en poids, par rapport au poids total du milieu de support anhydre.

6. Milieu de support anhydre pour la stabilité au stockage selon l'une des revendications 1 à 5, **caractérisé en ce que** l'alcane est un alcane en C10-C24, de préférence un alcane en C12-C18 et plus préférablement un alcane en C14-C16.

7. Agent cosmétique, comprenant un milieu de support anhydre selon l'une des revendications 1 à 6 ainsi qu'une phase aqueuse, **caractérisé en ce que** le milieu de support anhydre ou la phase aqueuse comprend un tensioactif cationique, de préférence un composé alkyltrimonium comportant un ou plusieurs groupes alkyle en C8-C22, plus préférablement en C10-C18, encore plus préférablement en C12-C16, ou dans lequel le tensioactif cationique est l'un de ceux de formule suivante, 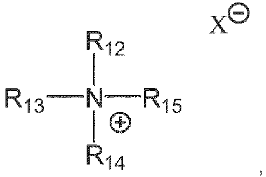 où
R₁₂, R₁₃, R₁₄ représentent, indépendamment les uns des autres, un groupe alkyle en C1-C6, un groupe alcényle en C2-C6 ou un groupe hydroxyalkyle en C2-C6,
R₁₅ représente un groupe alkyle en C8-C28, de préférence un groupe alkyle en C10-C22, et
X- représente un anion physiologiquement acceptable,
ou dans lequel le tensioactif cationique est l'un de ceux de formule suivante, 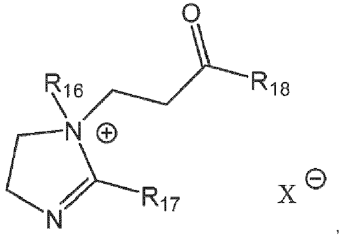 où
R₁₆ représente un groupe alkyle en C1-C6
R₁₇, R₁₈ représentent, indépendamment l'un de l'autre, un groupe alkyle en C7-C27, de préférence un groupe alkyle en C10-C22, et
X- représente un anion physiologiquement acceptable,
ou dans lequel le tensioactif cationique est l'un de ceux de formule suivante, 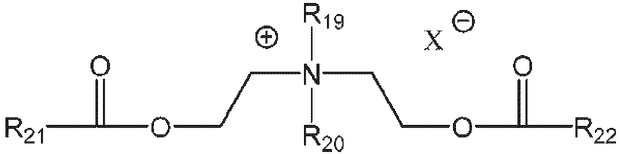 où
R₁₉, R₂₀ représentent, indépendamment l'un de l'autre, un groupe alkyle en C1-C6 ou un groupe hydroxyalkyle en C2-C6,
R₂₁, R₂₂ représentent, indépendamment l'un de l'autre, un groupe alkyle en C7-C27, de préférence un groupe alkyle en C10-C22, et
X- représente un anion physiologiquement acceptable,
ou dans lequel le tensioactif cationique est l'un de ceux de formule suivante,
NR₂₃R₂₄R₂₅,
où
R₂₃, R₂₄ représentent, indépendamment l'un de l'autre, un groupe alkyle en C1-C6, un groupe alcényle en C2-C6 ou un groupe hydroxyalkyle en C2-C6, et
R₂₅ représente un groupe alkyle en C8-C28, de préférence un groupe alkyle en C10-C22, et/ou
**caractérisé en ce que** le tensioactif cationique est contenu dans la phase aqueuse en une quantité allant de 0,1 à 5 % en poids, de préférence de 0,5 à 4 % en poids, plus préférablement de 1 à 3 % en poids, par rapport au poids total de la phase aqueuse.

8. Agent cosmétique selon la revendication 7, **caractérisé en ce que** le milieu de support anhydre ou la phase aqueuse comprend un tensioactif non ionique, dans lequel le tensioactif non ionique est de préférence un éthoxylate d'alcool gras, dans lequel la partie alcool gras de l'éthoxylate d'alcool gras présente une longueur de chaîne alkyle de C4-C30, de préférence de C6-C25, plus préférablement de C8-C20, et/ou dans lequel le nombre de groupes éthoxy dans l'éthoxylate d'alcool gras va de 2 à 120, de préférence de 4 à 100, plus préférablement de 6 à 80, encore plus préférablement de 8 à 60, le plus préférablement de 10 à 40.

9. Agent cosmétique selon la revendication 7 ou la revendication 8, **caractérisé en ce que** le milieu de support anhydre ou la phase aqueuse comprend un tensioactif anionique, dans lequel le tensioactif anionique est de préférence un sulfate d'alcool gras comportant une longueur de chaîne de C8-C22, de préférence de C10-C20, plus préférablement de C12-C18.

10. Agent cosmétique selon l'une des revendications 7 à 9,
**caractérisé en ce que** le rapport pondéral du milieu de support anhydre à la phase aqueuse se situe dans la plage allant de 1 pour 10 à 10 pour 1, de préférence de 5 pour 1 à 1 pour 5, plus préférablement de 2 pour 1 à 1 pour 2.
